# EUROPEAN PATENT APPLICATION

(11) **EP 4 707 783 A1**
(43) Date of publication of application: **11.03.2026**
(21) Application number: 25189115.6
(22) Date of filing: 11.07.2025
(51) Int. Cl.: G01N 21/47, G01N 21/77, G01N 21/94, G01N 33/543, G01N 35/00, G01N 21/01, G01N 33/02, G01N 33/18

(54) **APPARATUS FOR DETECTING TARGET ANALYTES IN A SAMPLE APPLIED ON A DIFFRACTIVE SENSOR**

(30) Priority: 06.09.2024 IT 202400019978
(71) Applicant: DG GROUP S.p.A., 28100 Novara (IT)
(72) Inventor: RADICE, Dino, 20010 Marcallo con Casone (MI) (IT); SILVESTRINI, Lucia, 20017 Rho (MI) (IT)
(74) Representative: Praxi Intellectual Property Milano

(57) **Abstract**

The present invention relates to an apparatus (100) for the detection of at least one target analyte in a sample to be analysed applied to a diffractive sensor (1), comprising:
- a housing (101) delimiting within it a dark chamber (102);
- a support device (105) suitable for supporting and integrally holding the diffractive sensor (1), said support device (105) being movable within the dark chamber (102) and between the inside and outside of the dark chamber (102);
- a source (103) of laser light suitable for emitting laser light with a wavelength within the visible spectrum in the darkroom (102);
- a screen (107) arranged in the dark chamber (102);
- a vision system (111) configured to capture images on the screen (107);
- a control unit operatively connected to the laser source (103), the support device (105) and the vision system (111).

## Description

### Technical field of the invention

The present invention relates to the field of detection of target analytes in a sample applied on diffractive sensors, in particular, an apparatus for analyzing diffraction images produced by such a diffractive sensor and subsequent determination of the presence or absence of the target analytes sought.

The term "target analyte" means any chemical species whose presence in the sample is to be determined.

The present invention finds in particular application for the detection of target analytes, such as viruses or bacteria or their components (e.g. nucleic acids, protein components, etc.), but can also be applied for the detection of target analytes of other kinds, thus not only in the medical, veterinary and diagnostic fields, but also for example in the biosafety or chemical fields, in particular for the detection of trace contaminants, as will be said in more detail below.

### Prior art

Diffractive sensors are known that can change a diffraction image produced when placed in contact with a sample containing a certain target analyte.

For example, with reference to Figure 1, European Patent Application No. 24164097.8, filed by Applicant, the contents of which are fully incorporated herein by reference, describes a diffractive sensor 1 for detecting a generic target analyte or a plurality of target analytes. The sensor 1 comprises, preferably on a transparent or semi-transparent support layer 2, a diffractive layer 3 having a diffractive grating 30 nanostructured, i.e., provided with diffractive structures having a depth on the order of a few tens to a few hundreds of nanometers. The diffractive layer 3 comprises a plurality of mutually equal surface regions, i.e., in which the diffractive grating 30 has the same conformation, in which the diffractive grating 30 has grooves forming a pattern having a random pattern, which is repeated equally in each surface region. A protective layer 4 can be provided to protect diffractive grating 30.

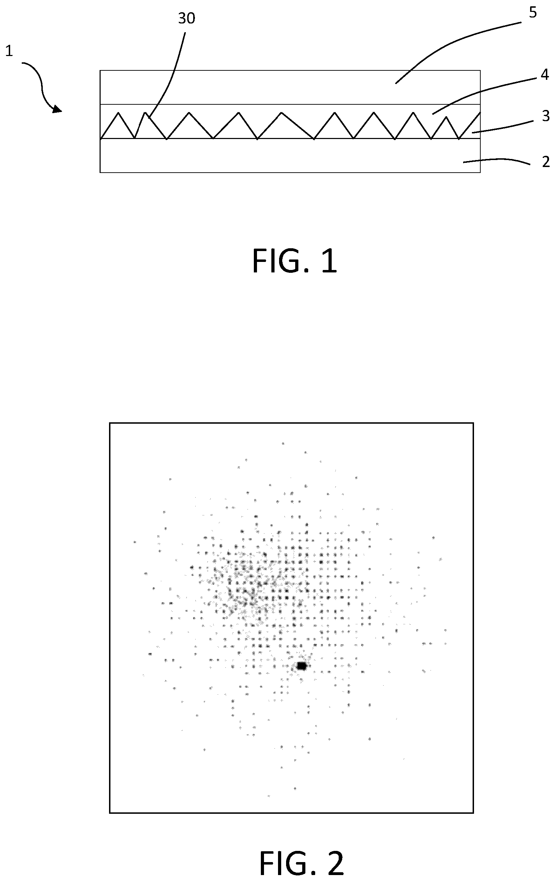

The diffractive grating 30 of the diffractive layer 3 causes a beam of monochromatic, polarized light (LASER) to pass through the sensor 1, such a beam of light is diffracted into a diffraction image visible to the naked eye, as shown for example in Figure 2. Such a diffraction image comprises a plurality of dots whose distribution depends on the conformation of the repeated diffractive grating 30 in the surface regions.

The diffractive sensor 1 also includes a receptor layer 5 superimposed on the diffractive layer 3. Receptor layer 5 is able to bind selectively to the target analyte to be detected and not to substances of a different nature. The target analyte may be contained in a sample, for example a clinical specimen such as a biological solution (e.g., a saliva or blood or urine sample), which may be deposited, e.g., smeared or smear, on receptor layer 5. The target analyte, rather than in solution, may alternatively be insoluble in the sample.

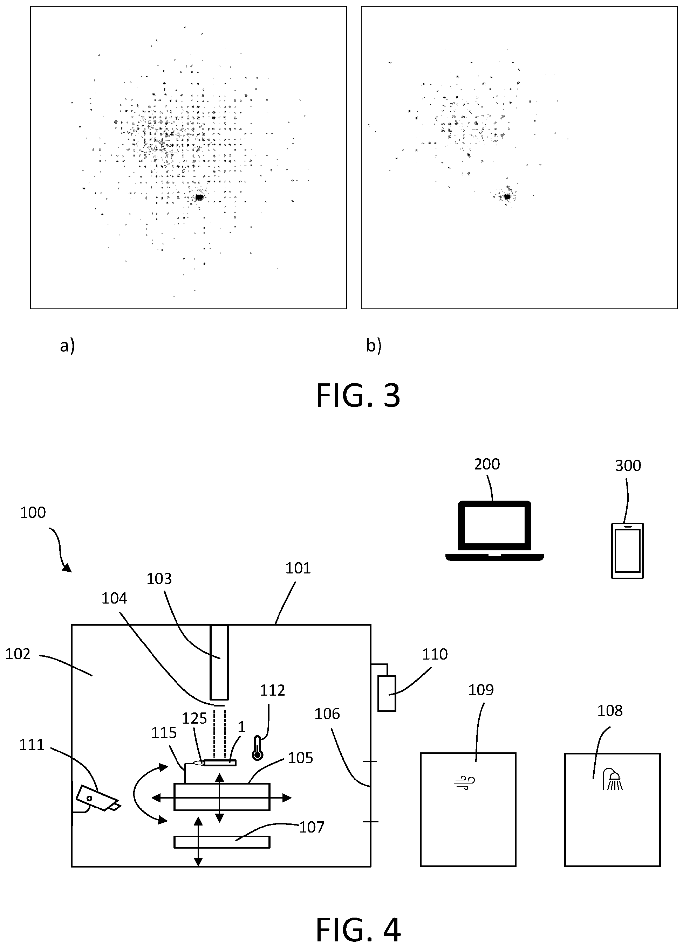

In this way, if the target analyte is not present in the sample, the receptor layer 5 is not altered and diffractive sensor 1, when subjected to a laser light beam, produces a reference diffraction image on the screen, visible to the naked eye. On the other hand, when the target analyte is present in the sample, it binds to receptor layer 5, and as a result, diffractive sensor 1 produces a verification diffraction image that is different from and comparable to the reference diffraction image, which is always visible to the naked eye. Figure 3 shows a comparison between a diffraction image produced in the case of absent target analyte (Figure 3(a)) and in the case of target analyte present and bound to receptor layer 5 (Figure 3(b)). As can be seen, the number and/or distribution and/or light intensity of the dots visible in the diffraction image of the first and second cases are different. Therefore, by comparing the two diffraction images, it is possible to determine whether the target analyte is present in the sample and whether it has bound to receptor layer 5 of diffractive sensor 1, or not.

In general, in the case where the target analyte is an antigen, receptor layer 5 may include the antibody specific for that antigen and unable to bind antigens other than the target antigen, which is intended to be detected by diffractive sensor 1. That antibody is firmly bound to diffractive layer 3.

Optionally, receptor layer 5 is capable of selectively binding to a plurality of different target analytes, such as in different areas of receptor layer 5, and is configured such that, depending on the target analyte bound and, possibly, its amount, the diffraction image produced is different. For example, receptor layer 5 may comprise different antibodies positioned such that receptor layer 5 is able to selectively bind to different target antigens.

### Brief summary of the invention

An object of the present invention is therefore to provide an apparatus for the detection of target analytes in a sample placed on a diffractive sensor , by way of example but not limitation a diffractive sensor of the type described in the above-mentioned European Patent Application No. 24164097.8, which enables such detection to be performed automatically or semi-automatically in a simple, fast and reliable manner.

This and other objects are achieved by an apparatus for detecting at least one target analyte in a sample applied to a diffractive sensor according to claim 1.

The dependent claims define possible advantageous embodiments of the invention.

### Brief description of the drawings

To better understand the invention and appreciate its advantages, some non-limiting exemplary embodiments of the invention will be described below, referring to the appended figures, in which:
Figure 1 is a schematic illustration of a diffractive sensor of the type described in European Patent Application No. 24164097.8;
Figure 2 shows a possible diffraction image produced by the diffractive sensor in Figure 1;
Figures 3 a) and b) show two possible diffraction images produced by the diffractive sensor in Figure 1, in the absence and presence, respectively, of a target analyte in a sample applied to the diffractive sensor itself;
Figure 4 is a schematic illustration of an apparatus for detecting target analytes in a sample applied on a diffractive sensor according to one possible embodiment of the invention;
Figure 5 is a perspective view of the apparatus for detecting target analytes in a sample applied to a diffractive sensor according to a possible constructive embodiment of the invention;
Figure 6 is a perspective view of the apparatus in Figure 5 partially disassembled.

### Detailed description of the invention

Referring to the appended Figure 4, an apparatus for detecting target analytes in a sample applied on a diffractive sensor 1, e.g., the diffractive sensor in Figure 1, is referred to collectively as reference 100.

The apparatus 100 comprises a housing 101, for example, conformed and sized as a box suitable for resting on a plane, such as on a table. The housing 101 delimits within it a dark chamber 102.

The apparatus 100 comprises a laser source 103, which may be arranged at least partially within the dark chamber 102, such that it is capable of emitting a beam of laser light within the dark chamber 102. As an example, the laser light emitted by laser source 103 can have wavelength λ equal to 532 nm (green light), but, more generally, laser source 103 can emit laser light having any wavelength in the visible spectrum (indicatively between 390 nm and 700 nm). Laser source 103 can optionally comprise a shutter 104 such that the size of the laser light beam can be changed.

The apparatus 100 further comprises a support device 105 suitable for supporting the diffractive sensor 1. Advantageously, the support device 105 is conformed such that it can hold, e.g., staple, reversibly, fixedly the diffractive sensor 1, such that the support device 105 and the diffractive sensor 1 are movable in a solid manner. For example, the support device 105 may comprise a slit 125 into which the diffractive sensor 1 can be inserted, e.g., cantilevered. The support device 105 is movable in translation (horizontally and/or vertically) within the dark chamber 102 and between the interior of the dark chamber 102 and its exterior, i.e., outside the housing 101. In addition, the support device 105 is movable upward and downward (vertically), so that the position of the diffractive sensor 1 can be changed with respect to the laser source 103 inside the dark chamber 102. According to one embodiment, the support device 105 is also rotatably movable, or comprises a rotatably movable portion 115, so as to change the spatial orientation of the diffractive sensor 1 integral to it. For example, the movable portion 115 may comprise the slit 125.

The laser source 103 and supporting device 105 are preferably spatially oriented such that the laser light beam strikes the diffractive sensor at 90°, although different beam angles are possible. Referring to the example in Figure 1, the laser light beam hits the outer surface of receptor layer 5, on which the sample is applied, preferably at 90°.

In order to allow the exit and entry of the support device 105 from/into the dark chamber 102, the housing 101 advantageously comprises a closable opening 106, possibly automatically.

The apparatus 100 further comprises, within the dark chamber 102, a screen 107 onto which the diffraction image of the diffractive sensor or 1 is projected when it is struck by the laser light beam emitted by the laser source 103. The screen 107 is preferably arranged so that the surface onto which the diffraction image is projected is oriented at 90° to the laser light beam. The screen 107 may, for example, be opaque and may comprise, for example, a polyester layer, preferably less than 500 µm thick, even more preferably less than 300 µm thick, e.g., equal to about 270 µm. Optionally, the screen 107 may comprise optical means for light amplification, to amplify the brightness of the diffraction image produced by the diffractive sensor, for example, one or more layers of photonic crystals. Advantageously, the distance of the screen 107 from the support device 105 is adjustable, either manually or automatically, in such a way as to optimize the focus of the diffraction image produced by the diffractive sensor 1 on the screen 107.

Preferably, with reference to the normal operating conditions of the apparatus 100, the laser source 103, the support device 105 and the screen 107 are arranged in this order from top to bottom in the dark chamber 102, as shown in Figure 4. Alternatively, different spatial orientations may be provided, as will become clear to the skilled person.

The apparatus 100 further comprises a vision system capable of capturing the diffraction image produced by the diffractive sensor 1. For example, the vision system may comprise a video camera 111 pointed at the screen 107. Alternatively, the vision system can be embedded in the screen 107, which can then itself acquire the diffraction image without the aid of a camera. Optionally, the vision system may be able to focus the image directly on the sensor.

According to one possible embodiment, the apparatus 100 comprises a system of thermoregulation 112 configured to keep the temperature inside the dark chamber 102 in the vicinity of the support device 105 within a predefined temperature range, for example, between 4°C and 40°C, particularly between 25°C and 35°C. Such a thermoregulation system 112 may comprise, for example, a temperature sensor and heating and/or cooling means.

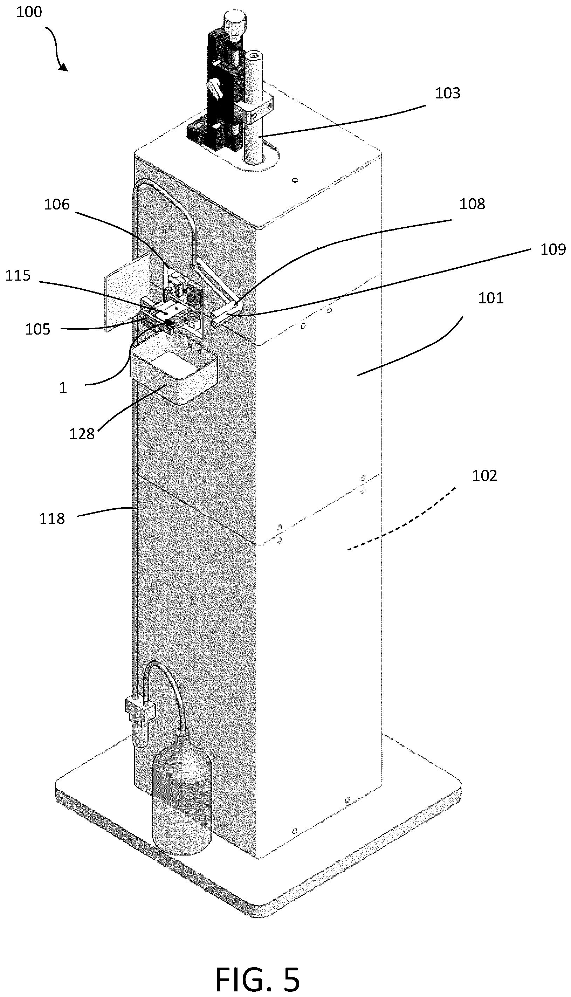

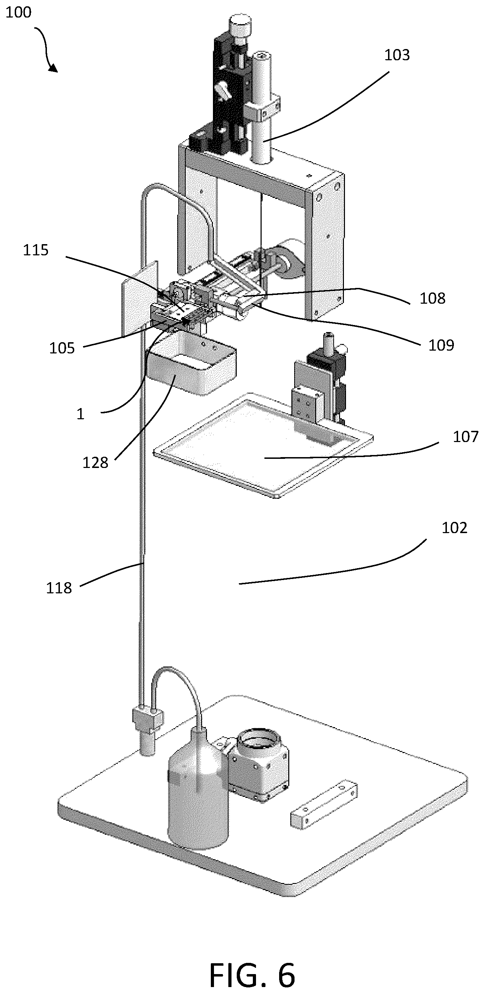

In accordance with an embodiment, the apparatus 1 comprises a device 108 for washing the diffractive sensor 1, preferably arranged outside the dark chamber 102. The washing of the diffractive sensor 1 is carried out following the application to it of the sample in which the target analyte is to be sought, for the purpose of removing substances or molecules or pollutants other than the target analyte from the receptor layer 5, which could interfere with the diffraction image produced, even if only as background noise. Washing can be done, for example, by the use of buffer solutions, ionic or nonionic detergents, or by mild surfactants. In cases where the receptor layer 5 includes antibodies or other proteins, washing can, for example, be carried out by the use of PBS (Phosphate Buffered Saline), possibly with the addition of mild detergents such as non-ionic surfactants, by immersion and possibly by subsequent centrifugation of the sensor. For this purpose, the washing device 108 may further comprise a suitable hydraulic circuit 118 for the supply and delivery of the detergent fluid, and, in addition, it may comprise a collection tank for residual detergent fluid 128 (in this regard, see Figures 5 and 6).

According to one possible embodiment, the apparatus 100 further comprises a device 109 for drying the diffractive sensor 1 following the previously mentioned washing. The drying device 109 can be separate from the washing device 108, or alternatively, be integrated into it, preferably outside the dark chamber 102. The drying device 109 may, for example, comprise a compressed air dispenser, optionally heated.

According to one embodiment, the apparatus 100 comprises a reader 110 configured to detect a code arranged on the diffractive sensor or a container thereof (e.g: a numeric or alphanumeric code, or a bar code, or a QR code) that identifies its characteristics and provides the necessary information about the analysis that can be performed, e.g., the types and quantities of target analytes that can be detected and, consequently, allows the apparatus 100 to move its parts and acquire the diffraction images in the appropriate manner to perform the search for the desired target analytes, according to what will be said in more detail below.

Apparatus 100 comprises a control unit operationally connected at least to the laser source 103, the supporting device 105 (and possibly its movable portion 115) and the vision system, and optionally to at least one of: the shutter 104, the closable aperture 106, the screen 107, the washing device 108, the drying device 109, the reader 110, and the temperature control system 112. The control unit is configured to govern automatic or semiautomatic operation of the apparatus 100. The control unit can be implemented by a hardware device (such as a control unit), by software, or by a combination of hardware and software. It may be integrated into apparatus 100 and connected to an external calculator 200, or alternatively, it may be part of the external calculator 200. The control unit and/or the calculator 200, provided with or connected to the control unit, may also be operatively connected to a portable device 300, such as a cell phone or smartphone, for example, via a data network, on which, for example, an application may be loaded for the remote control of the apparatus 100 and/or for making available sample analyses performed by the apparatus 100 itself. Computer 200 and/or portable device 300 can also serve as an interface between apparatus 100 and an operator.

The control unit is configured to, starting from a condition of support device 105 outside the dark chamber 102 and diffractive sensor 1 placed on it without application of the sample to be analyzed:
- receive information about diffractive sensor 1. This information can be entered manually by an operator, e.g., via the calculator 200 and/or via the portable device 300, or it can be detected automatically by the reader 110, where present, which can detect the code marked on the diffractive sensor 1 or its container and determine the information about the diffractive sensor from it;
- move the supporting device 105 with diffractive sensor devoid of the sample to be analyzed inside the dark chamber 102 and place it in a predefined position, determined, for example, on the basis of previously received information, relative to the laser source 103 and the screen 107. At this stage, the control unit can also command the opening 106 of the housing 101 to move from a closed position to an open position to allow the support device 105 with diffractive sensor 1 to pass through, and then return to the closed position following the passage thereof;
- command the laser source 103 to hit the diffractive sensor 1 on the support device 105 with a laser light beam with defined characteristics (e.g., with a certain wavelength and amplitude, which can be set by commanding the shutter 104) and command the vision system to detect a reference diffraction image projected by the diffractive sensor 1 in the absence of the sample on the screen 107. During the detection of the reference diffraction image, the control unit can further move the support device 105 and/or the screen 107 to change their relative distance and thus make a correct focus of the reference diffraction image on the screen 107;
- move the support device 105 with diffractive sensor 1 devoid of the sample to be analyzed to the outside of the dark chamber 102, for the sample to be analyzed to be applied to it. At this stage the control unit can again command the opening 106 of the housing 101 to move from a closed position to an open position, to allow the passage of the support device 105 with diffractive sensor 1, and then return to the closed position following the passage therof;
- control the washing device 108, if present, so that it washes the diffractive sensor 1 to remove any substances or molecules or contaminants other than the target analyte present on the diffractive sensor, e.g., anchored to receptor layer 5 of the diffractive sensor in Figure 1. Optionally, the control unit can be configured to rotate the support device 105 or its portion 115 so that the diffractive sensor is tilted, e.g., 90° (thus with the surface of receptor layer 5 placed vertically), during washing, thereby promoting the flow of wash residue and detergent solution. Depending on the positioning of the washing device 108, the control unit can further command the support device 105 to position itself at the washing device 108;
- command the drying device 109, if present, to carry out drying of the diffractive sensor 1 following washing. Depending on the positioning of the support device 105, the control unit can further command the drying device 109 to position itself at the support device. Optionally, the control unit is configured to rotate the support device back to its pre-wash orientation following drying (e.g., with the surface of receptor layer 5 horizontal);
- move the support device 105 with diffractive sensor 1 on which the sample to be analyzed is applied inside the dark chamber 102 and place it in the same default position relative to the laser source 103 and screen 107. At this stage, the control unit can again command the opening 106 of the housing 101 to move from a closed position to an open position to allow the support device 105 with diffractive sensor 1 to pass through, and then return to the closed position following the passage thereof;
- command the laser source 103 so that it strikes the diffractive sensor 1 with a beam of laser light with the same defined characteristics used for detecting the reference diffraction image, and command the vision system to detect a verification diffraction image produced by the diffractive sensor 1 with the sample applied. During the detection of the verification diffraction image, the control unit can further move the support device 105 and/or the screen 107 to change their relative distance and thus make a correct focus of the verification diffraction image on the screen 107;
- compare the verification diffraction image with the reference diffraction image. This comparison can be made, for example, by analyzing the dots in each diffraction image, comparing their number and/or distribution and/or light intensities;
- determine the presence of the target analyte in the sample if the verification diffraction image is different from the reference diffraction image.

In the event that the diffractive sensor 1 is able to selectively bind to a plurality of target analytes (information from the diffractive sensor, which may be provided to the control unit as said above, i.e., manually via the calculator 200 and/or the portable device 300, or automatically via the reader 110), the diffraction images produced in the presence of each such detectable target analyte will be different from each other as well as different from the reference diffraction image. In such a case, the control unit can be configured to compare the verification diffraction image produced by the diffractive sensor with the sample applied with a plurality of stored diffraction images (each corresponding to a specific target analyte and/or, optionally, to one or more of its defined quantities: i.e., for the same target analyte, a series of diffraction images is stored, each representative of a quantity or range of quantities of the target analyte in the sample), and determine the presence of a specific target analyte of the plurality of target analytes detectable by the diffractive sensor if the verification diffraction image produced by the diffractive sensor coincides with the diffraction image stored for that specific target analyte and, optionally, its specific quantity.

The control unit is preferably configured to move the support device 105 with diffractive sensor 1 again with sample outside the dark chamber 102 at the end of the analysis, for its removal from the support device 105. In this way, a new analysis of an additional sample can be performed in the same manner as described above.

Figures 5 and 6 illustrate a possible constructional embodiment of the apparatus 100. The numerical references of the elements given in these Figures correspond to those given in the schematic illustration of the apparatus in Figure 4.

The apparatus according to the present invention can find application in a variety of fields, non-limiting examples of which are given below:
- Diagnostics in medical and veterinary fields:
   o Diagnosis, by demonstration of the pathogen or its components, of bacterial (such as: Lyme disease, Brucellosis, Syphilis), viral (such as: HIV, Hepatitis A,B,C), fungal (or presence of pathogenic fungi, such as Candida, *Aspergillus*) and parasitic (such as: Filaria, Leptospira, Lehismania, Giardia, Trypanosomes) infectious agents;
   o detection of viruses (such as: Coronavirus, HIV, Hepatitis, Ebola, Norovirus, Influenza, West Nile, Zika, Pox, Dengue);
   o diagnosis, by demonstration of specific clinically validated markers, of autoimmune diseases, due to the abnormal production by the immune system of autoantibodies or antibodies linked to specific diseases (for example: autoantibodies that destroy insulin-producing cells in the pancreas in type I diabetes);
   o detection and quantification of hormones (for example: human chorionic gonadotropin (hCG), follicle-stimulating hormone (FSH), testosterone);
   o screening of donated blood (e.g., for detection of viral agents, such as HIV);
   o detection of drugs (e.g., amphetamines, cocaine);
   o detection of tumor markers (e.g., prostate-specific antigen (PSA) for prostate cancer diagnosis);
   o diagnostics in the veterinary field, for example: detection of etiological agents causing African swine fever, avian influenza, bovine parvovirus, canine adenovirus, coronavirus, equine infectious anemia, feline leukemia, etc.).
- DNA research;
- Non-diagnostic applications:
   o Biosecurity:
      ▪ Surveillance of epidemics;
      ▪ Testing for biological weapons/bioterrorism;
      ▪ Testing in cosmetics (e.g., testing for metal contamination);
   o Contaminant detection:
      ▪ Detection and screening of contaminants in food (eg: *Salmonella sp., Escherichia coli, Campylobacter sp., Staphylococcus aureus);*
      ▪ Detection and screening of environmental pollutants (e.g., detection of heavy metals in water or soil);
      ▪ Detection of pesticides;
      ▪ Gunpowder detection (e.g., nitrate detection by specific enzymes, such as nitrate reductase).

To the above provided description of the apparatus for detecting target analytes in a sample applied on a diffractive sensor the skilled person, in order to meet specific contingent needs, may make numerous additions, modifications, or substitutions of elements with functionally equivalent ones, without, however, departing from the scope of the appended claims.

## Claims

1. Apparatus (100) for the detection of at least one target analyte in a sample to be analysed applied to a diffractive sensor (1), comprising:
- a housing (101) delimiting within it a dark chamber (102);
- a support device (105) suitable for supporting and integrally holding the diffractive sensor (1), said support device (105) being movable within the dark chamber (102) and between the inside and outside of the dark chamber (102);
- a source (103) of laser light suitable for emitting laser light with a wavelength within the visible spectrum in the darkroom (102);
- a screen (107) arranged in the dark chamber (102);
- a vision system (111) configured to capture images on the screen (107);
- a control unit operatively connected to the laser source (103), the support device (105) and the vision system (111), configured to:
• starting from a condition of the support device (105) arranged outside the dark chamber (102) and the diffractive sensor (1) lacking the sample to be analysed positioned on the support device (105), move the support device (105) inside the dark chamber (102) and position it in a predefined position relative to the laser source (103) and the screen (107);
• command the laser source (103) to hit the diffractive sensor (1) lacking the sample to be analysed with a laser light beam with defined characteristics, so that the diffractive sensor (1) lacking the sample to be analysed, hit by the laser light beam, projects a reference diffraction image onto the screen (107), said reference diffraction image being visible to the naked eye and comprising a plurality of dots;
• command the vision system (111) to detect said reference diffraction image;
• move the support device (105) outside the dark chamber (102) for the application to the diffractive sensor (1) of the sample to be analysed;
• move the supporting device (105) with the diffractive sensor (1) with the applied sample to be analysed inside the dark chamber (102) and reposition it to said predefined position relative to the laser source (103) and the screen (107);
• command the laser source (103) to hit the diffractive sensor (1) with the applied sample to be analysed with a laser light beam with the same defined characteristics, so that the diffractive sensor (1) with the applied sample to be analysed, hit by the laser light beam, projects a verification diffraction image onto the screen (107), said verification diffraction image being visible to the naked eye and comprising a plurality of dots;
• compare the verification diffraction image with the reference diffraction image by comparing the number and/or distribution and/or light intensities of the plurality of dots of the verification diffraction image and the reference diffraction image;
• determine the presence of the at least one target analyte in the sample to be analysed if the verification diffraction image is different from the reference diffraction image.

2. Apparatus (100) according to claim 1, wherein the laser source (103) comprises a shutter (104) configured to change the size of the laser beam, said control unit being configured to control said shutter (104).

3. Apparatus (100) according to claim 1 or 2, wherein the control unit is configured to move the support device (105) within the dark chamber (102) such that the distance of the diffractive sensor (1) relative to the laser source (103) is adjusted.

4. Apparatus (100) according to any one of the preceding claims, wherein the housing (101) comprises a closable opening (106) for the passage of the support device (105) between the inside and the outside of the dark chamber (102), wherein the control unit is configured to control the opening (106) of the housing (101) to pass from a closed position to an open position during the passages of the support device (105) between the inside and the outside of the dark chamber (102).

5. Apparatus (100) according to any one of the preceding claims, wherein the vision system comprises a camera (111) pointed at the screen (107) or is integrated into the screen (107).

6. Apparatus (100) according to any one of the preceding claims, further comprising a thermoregulation system (112) operatively connected to the control unit and controlled to maintain the temperature within the dark chamber (102) in the vicinity of the support device (105) within a predetermined temperature range.

7. Apparatus (100) according to any one of claims from 1 to 6, further comprising a reader (110) configured to detect a code arranged on the diffractive sensor (1) or a packaging thereof, identifying characteristics and information of the diffractive sensor (1), wherein the control unit is operatively connected to the reader (110) to receive said characteristics and information of the diffractive sensor (1).

8. Apparatus (100) according to any one of claims from 1 to 6, wherein the control unit is operatively connected to a computer (200) and/or to a mobile device (300) and is configured to receive manually entered diffractive sensor characteristics and information from them.

9. Apparatus (100) according to claim 7 or 8, wherein the control unit is configured to determine said predefined position of the support device (105) relative to the laser source (103) and the screen (107) based on said characteristics and information of the diffractive sensor.

10. Apparatus (100) according to any one of the preceding claims, wherein the diffractive sensor (1) is configured to selectively bind to said at least one target analyte and the apparatus (100) further comprises a diffractive sensor washing device (108) operatively connected to the control unit, said control unit being further configured to control the washing device (108) so that it performs on the diffractive sensor (1), following the application thereon of the sample to be analysed, a washing for the removal of substances or molecules or contaminants other than the target analyte prior to the acquisition of the verification diffraction image.

11. Apparatus (100) according to the preceding claim, wherein the support device (105) is further rotatably movable or comprises a rotatably movable portion (115), wherein the control unit is further configured to rotate the support device (105) or said rotatably movable portion (115) thereof to tilt the diffractive sensor (1) before and/or during washing.

12. Apparatus (100) according to claim 10 or 11, further comprising a device (109) for drying the diffractive sensor (1) operatively connected to the control unit, said control unit being further configured to control the drying device (109) to perform a drying of the diffractive sensor (1) with the applied sample to be analysed following the washing.

13. Apparatus (100) according to any one of the preceding claims, wherein the control unit is further configured to move the support device (105) and/or the screen (107) to change their relative distance and perform a focusing of the reference diffraction image and/or the verification diffraction image on the screen (107) during their acquisition by the vision system.

14. Apparatus (100) according to any one of the preceding claims, wherein the diffractive sensor (1) is configured to selectively bind to a plurality of target analytes, and the control unit is further configured to:
• compare the verification diffraction image different from the reference diffraction image with a plurality of stored diffraction images, each corresponding to a specific target analyte of the plurality of target analy tes;
• determine the presence of a specific target analyte of the plurality of target analytes if the verification diffraction image matches the stored diffraction image for said specific target analyte.

15. Apparatus (100) according to any one of the preceding claims, wherein the control unit is further configured to:
• compare the verification diffraction image different from the reference diffraction image with a plurality of stored diffraction images, each corresponding to a specific range of target analyte amount;
• estimate the target analyte amount of the plurality of target analytes if the verification diffraction image matches the stored diffraction image for said specific range of target analyte amount.
